# EUROPEAN PATENT SPECIFICATION

(11) **EP 1 322 366 B1**
(45) Date of publication and mention of the grant of the patent: **18.01.2006**
(21) Application number: 01967543.8
(22) Date of filing: 21.09.2001
(51) Int. Cl.: A61M 16/00, A61M 16/08

(54) **BREATHING SYSTEM**
BEATMUNGSVORRICHTUNG
SYSTEME RESPIRATOIRE

(30) Priority: 06.10.2000 GB 0024497
(43) Date of publication of application: 02.07.2003
(73) Proprietor: The University of Manchester, Manchester M13 9PL (GB)
(72) Inventor: MEAKIN, George Harold, Manchester M30 9HF (GB)
(74) Representative: Allman, Peter John
(86) International application number: PCT/GB2001/004225
(87) International publication number: WO 2002/028461

(56) References cited:
- EP-A- 0 274 065
- US-A- 4 320 754
- US-A- 6 041 781

## Description

The present invention relates to breathing systems which can be used to deliver gases to a patient and to remove gases exhaled by the patient. In this context the patient may be a human being receiving treatment from medical staff or an animal receiving treatment from veterinary staff.

In one known system, generally described as the Jackson Rees T-piece system or Mapleson F system, a mask covers a patient's nose and mouth. The mask is connected via a conduit to one limb of a T-junction, a second limb of which receives a fresh gas supply and a third limb of which is connected to a reservoir tube leading to an open-tailed reservoir bag. The bag expands as the patient exhales and then contracts as the patient inhales. The bag therefore provides medical staff with a method of monitoring the breathing patterns of the patient. The bag also allows staff to ventilate a patient if breathing difficulties arise by partially occluding the open tail of the bag by hand and squeezing the bag.

One of the major drawbacks of this system is that exhaled gases leave the reservoir bag through the open tail into the atmosphere immediately adjacent to the patient. These gases are potentially dangerous. If the patient is anaesthetised, then epidemiological studies suggest that exposure to exhaled gases may adversely affect the health of operating theatre staff and their families. Even if the patient is not anaesthetised, exhaled gases may carry bacteria causing risks of infection. For these reasons it is desirable to collect and safely dispose of exhaled gases, rather than releasing such gases directly into the environment.

In the past, attempts have been made to devise scavenging systems to collect exhaled gases from the Jackson Rees T-piece, but these have all suffered from disadvantages. In most of these systems, the exhaled gases are fed into a scavenging system through a tube attached to the open-tailed bag. This leads to the system becoming somewhat unwieldy and increases the risk of exposing the airway to excessively high pressures by kinking either the collecting tube or the open tailed bag. It may also increase the risk of disconnection of the system.

EP0274065 discloses a disposable re-breathing canister for administering oxygen and anaesthesia to a patient. The canister comprises a housing for a scrubber, including a manifold plate and a rigid container, and a flexible liner disposed within the rigid container. The liner forms an imperforate barrier between the interior of the rigid container and the manifold plates. A scrubber disposed between the manifold plate and the liner chemically reacts with gases conducted to it through one of the pair of coaxial passages formed in the manifold plate. Reacted gases are then moved through a tortuous passage either back to the other one of the coaxial passages or removed through an exhaust port. The interior of the rigid container connects with the source of pressure that allows the flexible lining to be collapsed, thus forcing treated gases fresh oxygen and anaesthesia into the lungs of a patient.

It is an object of the present invention to provide a breathing system which obviates or mitigates some or all of the problems outlined above.

According to the present invention, there is provided a breathing system comprising a flexible inner reservoir bag having an exhaled gas inlet and a gas outlet, and a second flexible outer bag in which the inner bag is received, the gas outlet of the inner bag opening into the outer bag, and the outer bag having an exhaled gas outlet which in use is connected to a gas collector, the system further comprising a first conduit connected to a face mask communicating with the exhaled gas inlet of the inner reservoir bag, a second conduit connected to the exhaled gas outlet of the outer bag, and a third conduit which in use communicates with a fresh gas source, wherein the first and third conduits are interconnected adjacent the face mask.

The first conduit may be bundled together with a first portion of the second conduit. The first conduit may be enclosed within the first portion of the second conduit to form a coaxial tube.

Preferably the first and third conduits meet at a common junction in the form of a T-piece. The third conduit may be bundled together with the second portion of the second conduit. The third conduit may be enclosed within the second portion of the second conduit to form a coaxial tube. The first and second portions of the second conduit may also meet at a common junction formed by the T-piece.

A first end of the inner bag adjacent the exhaled gas inlet may be fixed in position relative to the outer bag, for example by being secured to an exhaled gas conduit extending through a conduit to which the outer bag is connected. The inner bag may then extend freely within the outer bag from the first end to a second end which defines the gas outlet of the inner bag. The gas outlet may be defined by an open extension of the second end of the inner bag. Alternatively, rather than having the inner bag extend freely within the outer bag, the end of the inner bag may be fixed in position relative to the outer bag, the gas outlet of the inner bag being defined by at least one aperture in the inner bag located in the end of the inner bag remote from the exhaled gas inlet. The remote end of the inner bag may include a closed extension which is secured to the outer bag.

The nature of a breathing system in accordance with the present invention will be familiar to medical staff, removing the need for training in its use and reducing the likelihood of mistakes. Its construction is such that the medical practitioner can easily institute hand ventilation while monitoring the patient's airway and respiratory movements. The breathing system can be constructed reasonably cheaply and may be disposable, thus reducing the risks of cross-infection between patients. While particularly suitable for paediatric anaesthetics the breathing system can also be used in adult anaesthetics, in intensive care units and in veterinary work.

Embodiments of the present invention will now be described, by way of example, with reference to the accompanying drawings, in which:
Figure 1 is a schematic representation of a prior art device;
Figure 2 is a schematic representation of a breathing system in accordance with a first embodiment of the present invention;
Figure 3 is a representation of the breathing system schematically illustrated in Figure 2;
Figure 4 is a representation of the breathing system of Figure 3 after the separation of coaxial components;
Figure 5 is a section through a coaxial T-piece used in the breathing system of Figures 3 and 4; and
Figure 6 is a schematic representation of a breathing system in accordance with a second embodiment of the present invention.

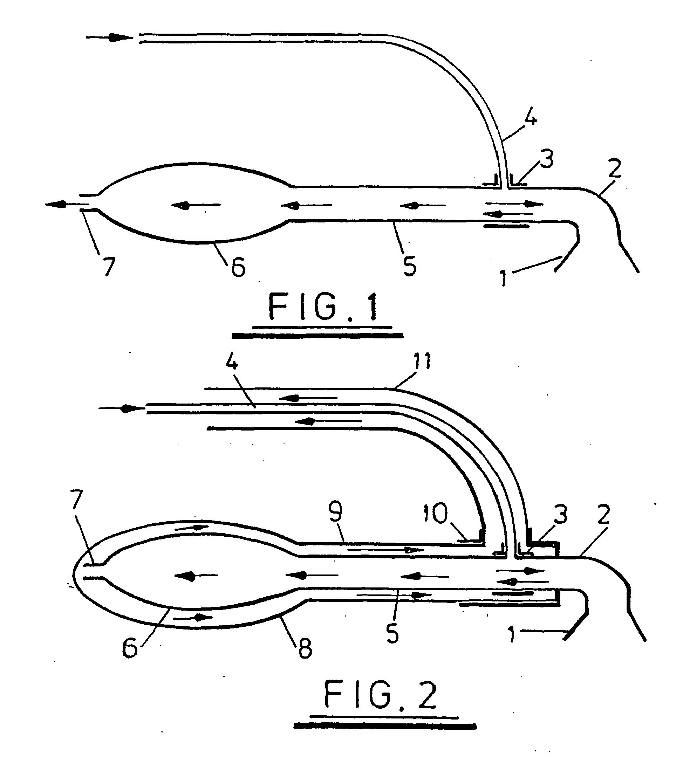

In Figures 1, 2, 5 and 6 arrows are used to denote the direction of flow of gas through the apparatus. Where appropriate, the same reference numerals are used in all the figures for equivalent components.

Referring to Figure 1, in the illustrated conventional (Jackson Rees T-piece) breathing system, a face mask 1 which may be positioned over a patient's nose and mouth is connected to a conduit 2 through which fresh and exhaled gases flow. A T-piece 3 connects conduit 2 to a conduit 4 having its other end attached to a fresh gas source. The third limb of the T-piece connects to a conduit 5 which has its other end attached to a reservoir bag 6. Exhaled gas leaves the reservoir bag through an extension to the bag 6 defining an open tail 7. In use, the bag 6 expands as the patient exhales, and contracts somewhat as the patient inhales.

Referring to Figure 2, in the illustrated embodiment of the invention the open tailed bag 6 is enclosed in a larger capacity outer bag 8. A conduit 9 carries exhaled gases from the bag 8 to an outer T-piece 10 which encloses the inner T-piece 3. Conduit 9 encloses conduit 5 to form a coaxial tube where conduit 5 is of smaller diameter. The end of the bag 6 though which gas enters is fixed in position relative to the outer bag 8 as a result of its connection to conduit 5 and the connection of the bag 8 to the conduit 9. The bag 6 extends freely from the conduit 5 across the interior of the bag 8 to the open tail 7. The T-piece 10 has one limb terminated and a third limb connected to a conduit 11 which carries exhaled gas to a scavenging system. Conduit 11 encloses conduit 4 to form a second coaxial tube.

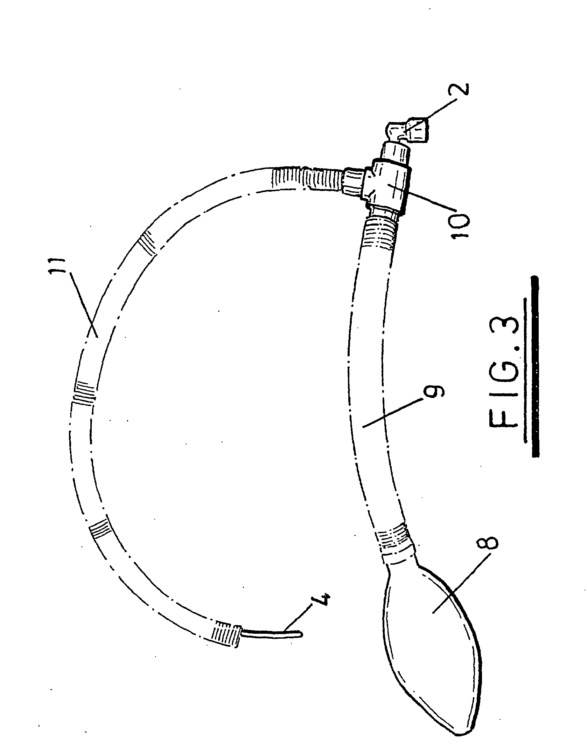

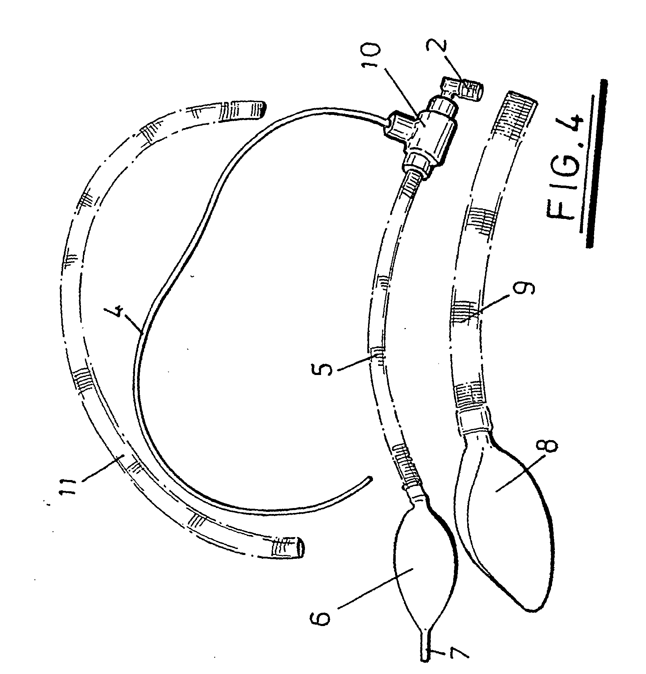

Referring to Figures 3 and 4, in the illustrated breathing system the conduit 2 is in the form of a standard adapter which may be used to make a connection to a standard mask (not shown). Fresh gas travels along conduit 4 which is enclosed within conduit 11. Exhaled gas is passed along conduit 5 to the inner bag 6 which when in use will be enclosed by the outer bag 8. Exhaled air leaves the outer bag along conduit 9, passes through the outer T-piece and passes along the conduit 11 to a scavenging system (not shown). Conduit 5 will be enclosed within conduit 9 when in use to form a coaxial tube.

The tail 7 of the inner reservoir bag 6 allows the inner bag to be sealed by the medical practitioner's hand, allowing ventilation of the patient if this is necessary. In use the inner bag 6 is enclosed within outer bag 8, the tail 7 being closed by applying pressure through the bag 8. Exhaled gas travels to the inner bag along conduit 5 and leaves the outer bag along conduit 9.

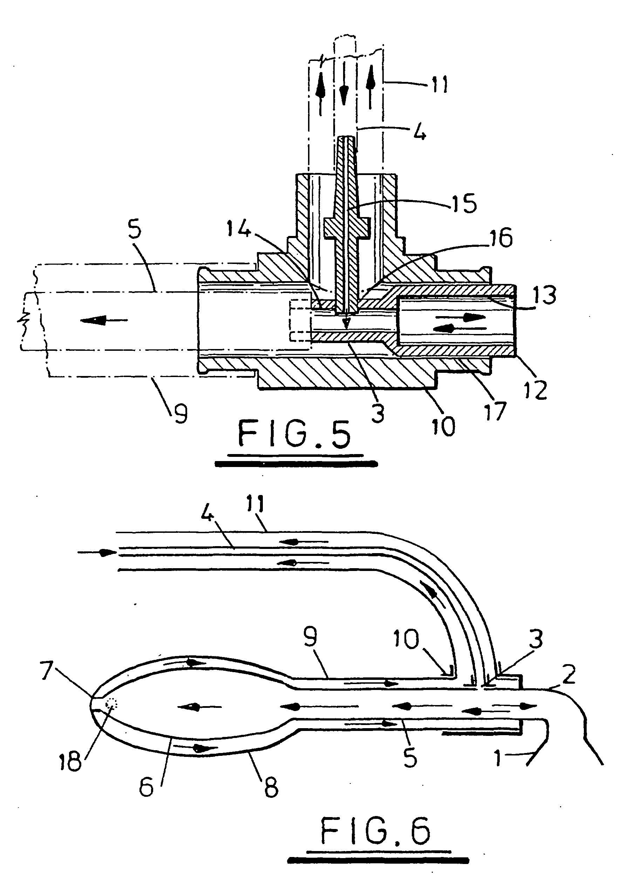

Figure 5 shows a coaxial T-piece defining the inner and outer T-pieces 10 and 3 which interconnect conduits 2, 4 and 5 and 9 and 11. The inner T-piece 3 comprises a first part 12 defining a cylindrical bore 13, a second part 14 communicating with but of smaller diameter than part 12, and a third part 15 which forms a T-junction at 16 with the second part 14. The outer T-piece 10 is a rigid assembly into which the inner T-piece 3 is inserted, the first part 12 of the inner T-piece 3 forming a tight fit in one limb 17 of the outer T-piece 10 to prevent air leakage.

A face mask may be attached to the first part 12 of the inner T-piece 3 using a standard adapter 2 shown in Figures 3 and 4 but not shown in Figure 5. The bore 13 is sized to receive such an adapter. Gases flow to the patient along conduit 4, pass through the inner T-piece 3, and through the adapter. Exhaled gases pass into the inner T-piece through the adapter, pass through the inner T-piece 3, and pass through conduit 5 to the inner bag 6. Exhaled gases returning from the outer bag 8 pass along conduit 9 into the outer T-piece 10 and leave through conduit 11. Only two limbs of the outer T-piece 10 are used, the third limb being sealed by the first part 12 of the inner T-piece 3.

In the embodiment of the invention described with reference to Figures 2 to 5, the inner bag 6 extends freely within the outer bag 8. To avoid any possibility of the bag 6 or the open tail 7 being folded over and thereby resulting in occlusion and distension of the inner bag 6, the inner bag 6 may be secured to the outer bag 8 so as to prevent folding. For example, as illustrated in Figure 6, the end of the bag 6 remote from the conduit 5 may be secured to the outer bag 8 at a point remote from the conduit 9. In the illustrated case, the tail 7 defined by the bag 6 is closed and secured to the bag 8. In order to permit venting of gas from the inner bag 6 to the outer bag 8, two 5mm diameter openings (one of which is represented by dotted line 18) are provided in the inner bag 6 adjacent the tail 7. Controlled ventilation may then be performed by compressing the inner and outer bags while partly occluding the vents in the inner bag.

## Claims

1. A breathing system comprising a flexible inner reservoir bag (6) having an exhaled gas inlet and a gas outlet (7), and a second flexible outer bag (8) in which the inner bag (6) is received, the gas outlet of the inner bag opening into the outer bag (8), and the outer bag having an exhaled gas outlet which in use is connectable to a gas collector, the system further comprising a first conduit (5) connected to a face mask (1) communicating with the exhaled gas inlet of the inner reservoir bag (6), a second conduit (9, 11) connected to the exhaled gas outlet of the outer bag (8), and a third conduit (4) which in use may communicate with a fresh gas source, **characterised in that** the first (5) and third (4) conduits are interconnected adjacent the face mask (1).

2. A breathing system according to claim 1, wherein the first conduit (5) is bundled together with a first portion of the second conduit (9).

3. A breathing system according to claim 2, wherein the first conduit (5) is enclosed within the first portion of the second conduit (9) to form a coaxial tube.

4. A breathing system according to claim 2 or 3, wherein the third conduit (4) is bundled together with a second portion of the second conduit (11).

5. A breathing system according to claim 4, wherein the third conduit (4) is enclosed within the second portion of the second conduit (11) to form a coaxial tube.

6. A breathing system according to any preceding claim, wherein the first and third conduits (5, 4) are interconnected by a T-piece (3).

7. A breathing system according to claim 6, wherein the second conduit (9, 11) communicates through a single assembly (10) incorporating the T-piece (3).

8. A breathing system according to claim 7, wherein the second conduit (9, 11) extends through a chamber which surrounds the T-piece (3).

9. A breathing system according to claim 8, wherein the second conduit extends through a further T-piece (10) one limb (17) of which is blocked.

10. A breathing system according to any preceding claim, wherein a first end of the inner bag (6) adjacent the exhaled gas inlet is fixed in position relative to the outer bag (8), and the inner bag extends freely within the outer bag from the first end to a second end (7) which defines the gas outlet of the inner bag.

11. A breathing system according to claim 10, wherein the gas outlet of the inner bag (6) is defined by an open extension of the second end of the inner bag (7).

12. A breathing system according to any one of claims 1 to 9, wherein a first end of the inner bag (6) adjacent the exhaled gas inlet is fixed in position relative to the outer bag (8), and the inner bag extends across the outer bag from the first end to a second end (7) which is fixed in position relative to the outer bag (8), the gas outlet of the inner bag being defined by at least one aperture (18) in the inner bag located adjacent the second end (7) of the inner bag (6).

13. A breathing system according to claim 12, wherein the second end (7) of the inner bag (6) includes a closed extension which is secured to the outer bag (8).

## Patentansprüche

1. Beatmungsvorrichtnng, die aufweist: einen elastischen inneren Behälterbeutel (6) mit einem Eintritt für ausgeatmetes Gas und einen Gasanstritt (7); und einen zweiten elastischen äußeren Beutel (8), in dem der innere Beutel (6) aufgenommen wird, wobei sich der Gasaustritt des inneren Beutels in den äußeren Beutel (8) öffnet, und wobei der äußere Beutel einen Austritt für ausgeatmetes Gas aufweist, der bei Benutzung mit einer Gassammelvorrichtung verbunden werden kann, wobei die Vorrichtung außerdem aufweist: einem ersten Kanal (5), der mit einer Gesichtsmaske (1) verbunden ist, die mit dem Eintritt für ausgeatmetes Gas des inneren Behälterbeutels (6) verbunden ist; einen zweiten Kanal (9, 11), der mit dem Austritt für ausgeatmetes Gas des äußeren Beutels (8) verbunden ist; und einen dritten Kanal (4), der bei Benutzung mit einer Frischgasquelle verbunden werden kann, **dadurch gekennzeichnet, dass** der erste (5) und der dritte Kanal (4) benachbart der Gesichtsmaske (1) miteinander verbunden sind.

2. Beatmungsvorrichtung nach Anspruch 1, bei der der erste Kanal (5) mit einem ersten Abschnitt des zweiten Kanals (9) zusammengebündelt ist.

3. Beatmungsvorrichtung nach Anspruch 2, bei der der erste Kanal (5) innerhalb des ersten Abschnittes des zweiten Kanals (9) eingeschlossen ist, um einen koaxialen Schlauch zu bilden.

4. Beatmungsvorrichtung nach Anspruch 2 oder 3, bei der der dritte Kanal (4) mit einem zweiten Abschnitt des zweiten Kanals (11) zusammengebündelt ist.

5. Beatmungsvorrichtung nach Anspruch 4, bei der der dritte Kanal (4) innerhalb des zweiten Abschnittes des zweiten Kanals (11) eingeschlossen ist, um einen koaxialen Schlauch zu bilden.

6. Beatmungsvorrichtung nach einem der vorhergehenden Ansprüche, bei der der erste und dritte Kanal (5, 4) mittels sines T-Stückes (3) miteinander verbunden sind.

7. Beatmungsvorrichtung nach Anspruch 6, bei der der zweite Kanal (9, 11) mittels einer einzelnen Baugruppe (10), die ein T-Stück (3) enthält, eine Verbindung herstellt.

8. Beatmungsvorrichtung nach Anspruch 7, bei der sich der zweite Kanal (9, 11) durch eine Kammer erstreckt, die das T-Stück (3) umgibt.

9. Beatmungsvorrichtung nach Anspruch 8, bei der sich der zweite Kanal durch ein weiteres T-Stück (10) erstreckt, von dem ein Schenkel (17) blockiert ist

10. Beatmungsvorrichtung nach einem der vorhergehenden Ansprüche, bei der ein erstes Ende des inneren Beutels (6) benachbart dem Eintritt für ausgestmetes Gas in Position relativ zum äußeren Beutel (8) befestigt ist, und bei der sich der innere Beutel ungehindert innerhalb des äußeren Beutels vom ersten Ende zu einem zweiten Ende (7) erstreckt, das den Gasaustritt des inneren Beutels definiert.

11. Beatmungsvorrichtung nach Anspruch 10, bei der der Gasaustritt des inneren Beutels (6) durch eine offene Verlängerung des zweiten Endes des inneren Beutels (7) definiert wird.

12. Beatmungsvorrichtung nach einem der Ansprüche 1 bis 9, bei der ein erstes Ende des inneren Beutels (6) benachbart dem Eintritt für ausgeatmetes Gas in Position relativ zum äußeren Beutel (8) befestigt ist und sich der innere Beutel über den äußeren Beutel vom ersten Ende zu einem zweiten Ende (7) erstreckt, das in Position relativ zum äußeren Bentel (8) befestigt ist, wobei der Gasanstritt des inneren Beutels durch mindestens eine Öffnung (18) im inneren Beutel definiert wird, die benachbart dem zweiten Ende (7) des inneren Beutels (6) angeordnet ist.

13. Beatmungsvorrichtung nach Anspruch 12, bei der das zweite Ende (7) des inneren Beutels (6) eine geschlossene Verlängerung umfasst, die am äußeren Beutel (8) gesichert ist.

## Revendications

1. Système respiratoire comprenant un sac réservoir interne flexible (6), comportant un orifice d'entrée des gaz exhalés et un orifice de sortie des gaz (7), et un deuxième sac externe flexible (8), dans lequel est reçu le sac interne, (6), l'orifice de sortie de gaz du sac interne étant ouvert vers le sac externe (8) et le sac externe comportant un orifice de sortie des gaz exhalés pouvant être connecté en service à un collecteur de gaz, le système comprenant en outre un premier conduit (5) connecté à un masque facial (1) communiquant avec l'orifice d'entrée des gaz exhalés du sac réservoir interne (6), un deuxième conduit (9, 11) connecté à l'orifice de sortie des gaz exhalés du sachet externe (8) et un troisième conduit (4) pouvant communiquer en service avec une source de gaz frais, **caractérise en ce que** les premier (5) et troisième (4) conduits sont interconnectés près du masque facial (1).

2. Système respiratoire selon la revendication 1, dans lequel le premier conduit (5) est relié à une première partie du deuxième conduit (9).

3. Système respiratoire selon la revendication 2, dans lequel le premier conduit (5) est renfermé dans la première partie du deuxième conduit (9) pour former un tube coaxial.

4. Système respiratoire selon les revendications 2 on 3, dans lequel le troisième conduit (4) est relié à une deuxième partie du deuxième conduit (11).

5. Système respiratoire selon la revendication 4, dans lequel le troisième conduit (4) est renfermé dans la deuxième partie du deuxième conduit (11) pour former un tube coaxial.

6. Système respiratoire selon l'une quelconque des revendications précédentes, dans lequel les premier et troisième conduits (5, 4) sont interconnectés par une pièce en T (3).

7. Système respiratoire selon la revendication 6, dans lequel le deuxième conduit (9, 11) communique à travers un seul assemblage (10) incorporant la pièce en T (3).

8. Système respiratoire selon la revendication 7, dans lequel le deuxième conduit (9, 11) s'étend à travers une chambre entourant la pièce en T (3).

9. Système respiratoire selon la revendication 8, dans lequel le deuxième conduit s'étend à travers une pièce en T additionnelle (10), dont un membre (17) est bloqué.

10. Système respiratoire selon l'une quelconque des revendications précédentes, dans lequel une première extrémité du sac interne (6) adjacente à l'orifice d'entrée des gaz exhalés est fixée dans sa position par rapport au sac externe (8), le sac interne s'étendant librement dans le sac externe, de la première extrémité vers une deuxième extrémité (7) définissant l'orifice de sortie des gaz du sac interne.

11. Système respiratoire selon la revendication 10, dans lequel l'orifice de sortie des gaz du sac interne (6) est défini par une extension ouverte de la deuxième extrémité du sac interne (7).

12. Système respiratoire selon l'une quelconque des revendications 1 à 9, dans lequel une première extrémité du sac interne (6) adjacente à l'orifice d'entrée des gaz exhalés est fixée dans sa position par rapport au sac externe (8), le premier sac s'étendant à travers le sac externe de la première extrémité vers une deuxième extrémité (7) fixée dans sa position par rapport an sac externe (8), l'orifice de sortie des gaz du sac interne étant défini par au moins une ouverture (18) dans le sac interne agencée près de la deuxième extrémité (7) dn sac interne (6).

13. Système respiratoire selon la revendication 12, dans lequel la deuxième extrémité du sac interne (6) englobe une extension fermée fixée sur le sac externe (8),
